(19) Europäisches Patentamt
European Patent Office
Office européen des brevets

(11) **EP 2 606 845 B1**

(12) **EUROPEAN PATENT SPECIFICATION**

(45) Date of publication and mention
of the grant of the patent:
**26.10.2016 Bulletin 2016/43**

(51) Int Cl.:
*A61B 18/12* *(2006.01)*   *A61B 18/14* *(2006.01)*

(21) Application number: **11195633.0**

(22) Date of filing: **23.12.2011**

(54) **Pulse generator**

Impulsgeber

Générateur d'impulsions

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(43) Date of publication of application:
**26.06.2013 Bulletin 2013/26**

(73) Proprietor: **Lina Medical ApS**
**2600 Glostrup (DK)**

(72) Inventor: **Poulsen, Henrik Bisgaard**
**3550 Slangerup (DK)**

(74) Representative: **Inspicos P/S**
**Kogle Allé 2**
**2970 Hørsholm (DK)**

(56) References cited:
**EP-A2- 2 160 993          WO-A1-2010/108523**
**WO-A2-2011/150200     US-A1- 2010 298 761**

## Description

The present invention relates to an electrosurgical instrument for use with an electrosurgical generator.

When using an electrosurgical instrument to coagulate tissue by electrical heating, parts of the body not supposed to be coagulated, but close to the tissue to be coagulated, is heated above a temperature where damage and necrosis is caused. To overcome this problem the electrical current is pulsed, which reduces the thermal spread but still coagulates the tissue to be coagulated. But another problem arises since the electrosurgical instrument must be connected to a generator that pulses the current. Actually, most electrosurgical generators are customised for use with a limited number of electrosurgical instruments. Other electrosurgical generator models cannot be used instead, and hospitals and medical/surgical performers are compelled to buy new and/or additional equipment to benefit from the advantages of developing technology within electrosurgical instruments. To have another generator in the surgical laboratory increases the danger that the physician or the nurse in a stressful situation does not find the right generator.

EP 2 160 993 illustrates an instrument 10 connected to a generator 20, which generator 20 includes a controller 24, a power supply 27, an RF output stage 28, and a sensor module 22. The power supply 27 may provide DC power to the RF output stage 28 which then converts the DC power into RF energy and delivers the RF energy to the forceps 10, but the generator is a separate component and the DC to RF conversion is inside the separate generator 20.

WO 2010/108523 shows a high frequency generator with a connected electrical instrument. The generator 10 is provided at the input side with a power cable for connection to a public mains system. The high frequency generator 10 is connected to a foot switch 240 serving to switch the high frequency generator 10 on and off. On the output side two output poles 125 of the high frequency generator 10 are connected by way of current lines to an electrode 210 of an electrosurgical instrument and a neutral electrode 220. The electrosurgical instrument in this case has a cutting electrode 210 which in the embodiment by way of example as shown in Figure 1 is in the form of a snare loop cutting instrument. I.e. in D2, the instrument is completely separate from the generator 10.

It is a main aspect of the present invention to provide an electrosurgical instrument that can be used with various conventional and/or novel electrosurgical generators.

In a second aspect according to the present invention is provided an electrosurgical instrument that can be used with any RF generator.

In a third aspect according to the present invention is provided an electrosurgical instrument that is economically attractive to use.

In a fourth aspect according to the present invention is provided an electrosurgical apparatus comprising an electrosurgical instrument and an electrosurgical generator that comply with standard safety regulations.

In a fifth aspect according to the present invention is provided an electrosurgical instrument that is less susceptible to unintended injury of tissue in the vicinity of the electrodes than known electrosurgical instruments.

In a sixth aspect according to the present invention is provided an electrosurgical instrument that is easy to handle.

The novel and unique whereby these and other aspects are achieved according to the present invention consists in the fact that the electrosurgical instrument incorporates a signal modificator having an input end electrically connected to the electrosurgical generator and an output end electrically connected to one or more electrodes of the electrosurgical instrument, where the signal modificator is adapted to modify a continuous signal from the electrosurgical generator into a pulsing signal to the one or more electrodes of the electrosurgical instrument and comprises a circuit for providing the pulsing signal.

The electrosurgical generator is generally a RF generator that can transmit an electrical current to the electrodes of the electrosurgical instrument to cut or coagulate tissue situated between the electrodes.

The signal modificator interrupts and transmits a signal from the electrosurgical generator to the one or more electrodes of the electrosurgical instrument with an even or an arbitrary interrupting and transmitting frequency or period. The signal modificator is pulsing the current from the electrosurgical generator. When coagulating tissue by driving an electrical current through the tissue also surrounding tissue that is not supposed to coagulate can be damaged due to increased temperature. By pulsing the current the thermal spread is reduced while the time to coagulate the tissue between the electrodes increases compared to when the coagulating current is not pulsed.

The circuit can be a printed circuit board. The circuit can be made very small and even so small that the circuit can be placed inside the handle of the electrosurgical instrument. There are prejudices within the art against using electronic circuits in instruments to be sterilised, since sterilisation by gamma rays, which is commonly used to sterilise medical equipment, destroys the electronics. Using ethylene oxide can also destroy the electronics and cause batteries to explode. In the present invention, though, the electrosurgical instrument is sterilised using ethylene oxide in a cycle that does not destroy the electronics and that is safe concerning the battery.

The signal modificator is incorporated inside a handle or part of a handle of the electrosurgical instrument.

Using the electrosurgical instrument comprising the signal modificator means that the electrosurgical instrument can be used together with any electrosurgical generator. The electrosurgical generator does not need to comprise a signal modificator. The advantage is that when buying an electrosurgical instrument that can pulse the current from the electrosurgical generator it is not necessary to buy a special electrosurgical generator comprising the signal modificator but any electrosurgical generator can be used that is suitable for coagulating tissue. An extra electrosurgical generator is not needed that will take space in the operating room and will need to be cleaned and kept free of dust.

In an expedient embodiment according to the present invention, the circuit can comprise a timer IC such as a 555 Timer IC, a Schmitt trigger, a Blocking oscillator, or an Armstrong oscillator.

The timer IC is a small electrical component known to the person skilled in the art. The timer IC is an integrated circuit that can create an oscillating signal.

In a favourable embodiment the circuit can control switching means to connect/disconnect the one or more electrodes to/from the electrosurgical generator.

The signal from the electrosurgical generator can be pulsed by the frequency of the disconnection. By pulsing the current the thermal spread is reduced while the coagulation of the tissue between the electrodes is nearly as good as when the coagulating current is not pulsed. Thus the risk, that tissue in the vicinity of the electrodes is unintentionally injured, is significantly reduced but not to the detriment of the efficiency of the electrosurgical instrument.

In a useful embodiment the signal modificator can be detachably secured to the electrosurgical instrument, optionally the circuit can be integrated in the handle or a part of the handle, optionally said handle or part of the handle can be detachable.

If the electrosurgical instrument is a disposable unit that is only used once and then thrown away it is an advantage that the signal modificator or the handle comprising the circuit is detachable since then the signal modificator or the handle can be sterilized again and the waste will be reduced.

Advantageously, the pulsing signal can have an interrupting frequency of between 0.1 and 10 Hz, preferably between 0.2 and 5 Hz, more preferably between 0.25 and 4 Hz, even more preferably between 0.33 and 3 Hz, further more preferably between 0.5 and 2 Hz, even further more preferably between 0.67 and 1.5 Hz and most preferably between 0.8 and 1.25 Hz.

A pulsing frequency between 0.1 and 10 Hz can be very useful but the closer to 1 Hz the better since the range from 0.8 Hz to 1.25 Hz gives the optimal relation between speed of the coagulation of the tissue between the electrodes and the reduction in thermal spread to tissue not to be treated.

Preferably, the electrosurgical instrument can comprise means to vary the interrupting frequency, thereby enabling the surgeon to optimise the frequency so that most optimal frequency is achieved for a particular surgery. To use the timer IC, such as the 555 Timer IC, the Schmitt trigger, or the Blocking oscillator, to create an oscillating signal, the timer IC is connected to resistors and capacitors, which resistances and capacitances, respectively, determines the oscillating frequency. The person skilled in the art knows how to connect the resistors and capacitors to the 555 Timer IC, the Schmitt trigger, or the Blocking oscillator. By using one or more potentiometers as the one or more resistors that control the frequency and/or one or more variable capacitors as the one or more capacitors that control the frequency, the frequency can by varied and optimised.

In another preferred embodiment, the pulsing signal can have a period comprising a pulse part and a quiet part, where the pulse part constitutes between 10% and 90%, preferably between 20% and 80%, more preferably between 30% and 70%, even more preferably between 40% and 60% and most preferably between 45% and 55% of the total period and the quite part constitutes the rest of the period.

In most situations the optimal ratio between the time when the signal from the electrosurgical generator is transmitted to the electrodes, and the time when the signal from the electrosurgical generator is interrupted is 1. The coagulation is fast while the surrounding tissue is not damaged.

In another embodiment the pulsing signal can have a period comprising a pulse part and a quiet part, where the pulse part constitutes between 50% and 95%, preferably between 55% and 90%, more preferably between 60% and 85%, even more preferably between 65% and 80% and most preferably between 65% and 75% of the total period and the quite part constitutes the rest of the period.

If the tissue to be coagulated is thick the tissue surrounding the tissue to be coagulated will not easily be damaged during the coagulation. During such conditions the ratio between the time when the signal from the electrosurgical generator is transmitted to the electrodes, and the time when the signal from the electrosurgical generator is interrupted can be above 1, preferably between 1.2 and 9, more preferably between 1.5 and 5.7, even more preferably between 1.8 and 4, and most preferably between 1.8 and 3. The coagulation will be achieved faster and the surrounding tissue will not be damaged anyway.

In yet another embodiment the pulsing signal can have a period comprising a pulse part and a quiet part, where the quiet part constitutes between 50% and 95%, preferably between 55% and 90%, more preferably between 60% and 85%, even more preferably between 65% and 80% and most preferably between 65% and 75% of the total period and the pulse part constitutes the rest of the period.

If the tissue to be coagulated is thin the tissue surrounding the tissue to be coagulated will easily be damaged during the coagulation. During such conditions the ratio between the time when the signal from the electrosurgical generator is transmitted to the electrodes and the time when the signal from the electrosurgical generator is interrupted can be below 1, preferably between 0.11 and 0.82, more preferably between 0.18 and 0,67, even more preferably between 0.25 and 0.54, and most preferably between 0.33 and 0.54. The coagulation may take some time but the surrounding tissue will not be damaged.

In a further advantageous embodiment the electrosurgical instrument can be configured to provide at least one of the functions cut, coagulate, and ablate.

It is an advantage that the electrosurgical instrument cannot only coagulate tissue by a pulsed signal but can also easily change to a mode where the tissue is cut or ablated by a non-pulsing signal.

In still another embodiment the electrosurgical instrument can be a pair of bipolar forceps, a bipolar loop electrode, a pair of bipolar scissors, a bipolar pencil, a bipolar scalpel, or a bipolar electrosurgical instrument incorporating and combining one or more functions thereof, or a monopolar pencil or scalpel.

The pulsed generator is very suitable together with a pair of bipolar forceps. The tissue to be coagulated is situated between the pair of forceps. This tissue will be held in a firm grip and cannot slip so that other parts of the tissue by accident are coagulated. Advantageously, the pair of bipolar forceps is combined with a cutting member that can protrude, e.g. in a reciprocating manner, between the forceps jaws and cut off the tissue before, during or after coagulation as occasion requires.

A bipolar loop electrode can be isolated except at the tip of the loop electrode. The other electrode or pole can be return electrodes at the base of the loop electrode. The loop electrode can also be parted in two so that one half of the loop is one electrode and the other half of the loop is the other electrode. The two halves of the loop are kept together at the tip of the loop so that the two halves are more or less equally large. With the bipolar loop electrode the user can alternately coagulate and cut the tissue. The bipolar pencil is particularly preferred when coagulating a tissue surface.

If tissue sticks to the surface of the electrodes some of the power to coagulate, cut or ablate is used to heat the tissue that sticks and less energy will be used to coagulate the tissue that is supposed to be coagulated. If tissue sticks to the surface of the electrodes the coagulation will take longer time and the thermal spread will be higher. To avoid these disadvantages at least one of the one or more electrodes of the electrosurgical instrument can have an area provided with a non-stick coating.

The invention will be described in further details below with reference to the accompanying drawings illustrating exemplary embodiments that shall not be construed as limiting the scope of the appended claims.

The invention is defined in the claims.

Other embodiments are merely exemplary.

Fig. 1 shows a principle sketch of an electrosurgical instrument with a printed circuit board,

fig. 2 shows a circuit diagram of the printed circuit board in fig. 1,

fig. 3 shows in an enlarged scale fragmentary view the first printed circuit board of the bipolar electrosurgical loop instrument shown in fig. 1, with the electrical components disclosed.

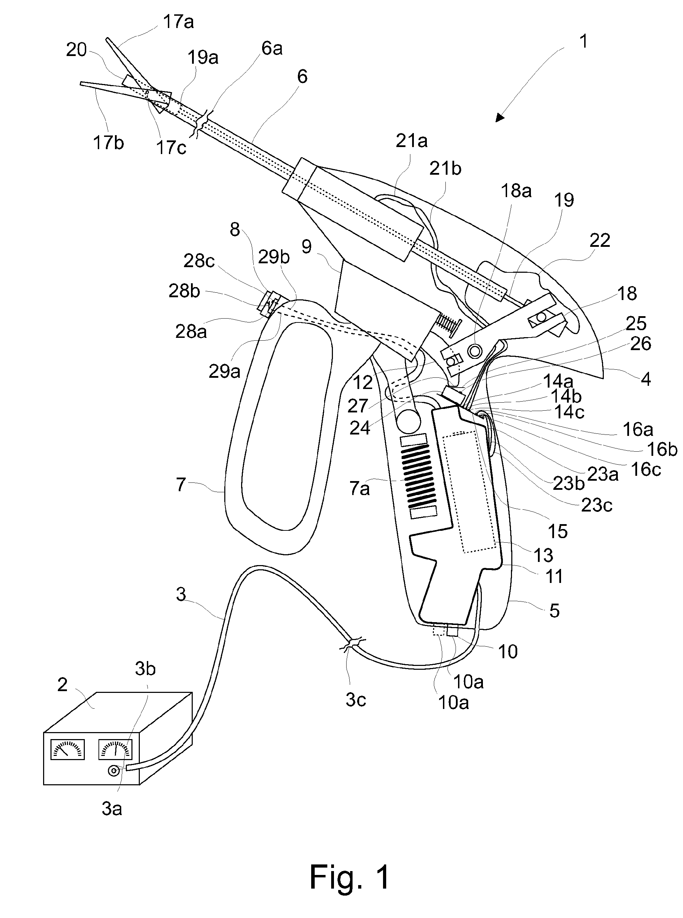

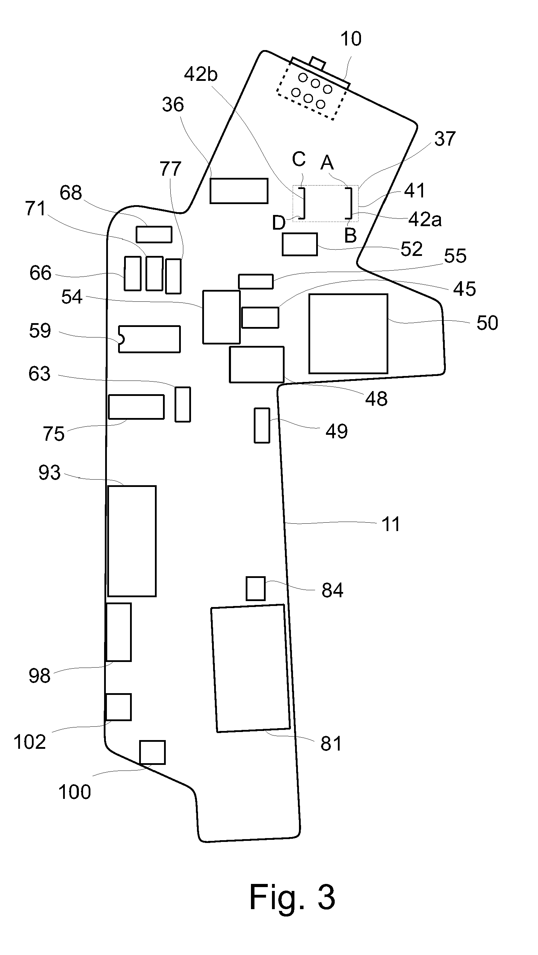

Fig. 1 shows an electrosurgical instrument 1 electrically connected to a generator 2 via an electric cable 3 including at least two electric wires 3a, 3b, where the electric wire 3a is the live connection or the phase conductor 3a and the electric wire 3b is the neutral connection or return connection 3b. The electrosurgical instrument 1 comprises a body 4 with a handle 5, a hollow shaft 6, a movable handle 7, a coagulation button 8, a cutting button 9, a slide switch 10, and a first printed circuit board 11. The slide switch 10 is positioned on and electrically connected to the first printed circuit board 11. The slide switch 10 and the first printed circuit board 11 are positioned in the handle 5. The slide switch 10 has an on-position 10a and an off-position 10b. The details of the first printed circuit board 11 are shown in fig. 3. The movable handle 7 is biased by a spring 7a. Behind the first printed circuit board 11 a battery 13, advantageously an AAA battery, is situated.

In fig. 1 the electric cable 3 and the hollow shaft 6 are for illustrative purposes shown fragmented by fragment lines 3c and 6a, respectively.

At the distal end of the hollow shaft 6 seen from the body 4 a first jaw 17a and a second jaw 17b are attached to the hollow shaft 6. The jaws 17a,17b pivot around an axis 17c when the movable handle 7 is moved towards the handle 5. The movable handle 7 activates means that presses the jaws 17a,17b together using e.g. a concept as described in European patent EP 1150616 or a scissor concept as described in US patent US 5,478,347.

The cutting button 9 through a protruding member 12 rotates a rocker arm 18 around a pivot axle 18a so that a rod 19 is pushed forward inside the hollow shaft 6. A cutting member 20 is attached to and positioned at a distal end 19a of the rod 19. When the rod 19 is pushed forward the cutting member 20 will also move forward and cut tissue secured between the jaws 17a,17b.

The generator 2 is a RF-generator supplying an RF-voltage or an RF-signal to the first printed circuit board 11 and the electrosurgical instrument 1. The first printed circuit board 11 can pulse the RF-signal. The pulsed RF-signal can be applied over the jaws 17a,17b for coagulating tissue or the non-pulsed RF-signal can be applied over the jaws 17a,17b on the one side and the cutting member 20 on the other hand for cutting the tissue.

The jaws 17a,17b are electrically connected via electric wires 21a,21b to a first output 14a and a second output 14b of a second printed circuit board 15. The cutting member 20 is electrically connected via electric wire 22 to a third output 14c of the second printed circuit board 15. The second printed circuit board 15 has three electrical inputs 16a,16b,16c to electrically connect to the first printed circuit board 11 via electric wires 23a,23b,23c.

The electric wire 23a supplies the pulsed RF-signal that is the RF-signal from the generator 2 pulsed by the first printed circuit board 11. The electric wire 23b supplies the non-pulsed RF-signal from the generator 2. The electric wire 23c is electrically connected to the neutral connection or return connection 3b.

The second printed circuit board 15 comprises a knife switch 24. The knife switch 24 has a contact member 25, which in a relaxed position bends away from an electrical contact area 26 on the knife switch 24. The relaxed position of the contact member 25 is shown in fig. 1. When the cutting button 9 is pressed down a distal end 27 of the protruding member 12 will press the contact member 25 in electrical contact with the electrical contact area 26.

When the coagulation button 8 is pressed down the coagulation button 8 pushes on a knife switch 28a so that a contact member 28b is pressed into electrical contact with an electrical contact area 28c and electrical wires 29a,29b are electrically connected to each other. The electrical connection between the electrical wires 29a,29b activates the first printed circuit board 11.

When the slide switch 10 is in the on-position 10a and the coagulation button 8 is pressed down while the cutting button 9 is not pressed down the voltage from the RF generator 2 is pulsed by the first printed circuit board 11 and is applied via the electric wires 23a,23c and directed by the knife switch 24 to be applied via the electric wires 21a,21b over the first and the second jaws 17a,17b.

When the cutting button 9 is not pressed down the knife switch 24 electrically connects electric wire 23a to the electric wire 21a and electric wire 23c to the electric wire 21b or vice versa.

When the slide switch 10 is in the on-position 10a while the cutting button 9 is pressed down and pressing the contact member 25 in electrical contact with the electrical contact area 26, the knife switch 24 is changed to electrically connect electric wire 23b to the electric wire 22 and electric wire 23c to the electric wires 21a and 21b. Then the non-pulsed RF-voltage from the RF generator 2 is applied via the first printed circuit board 11, via electric wires 23b,23c and directed by the knife switch 24 to be applied via the electric wires 21a,21b and the electric wire 22 on the two jaws 17a,17b and the cutting member 20. The neutral connection or return connection from the RF-generator 2 is electrically connected to the two jaws 17a,17b and the RF-signal is applied on the cutting member 20.

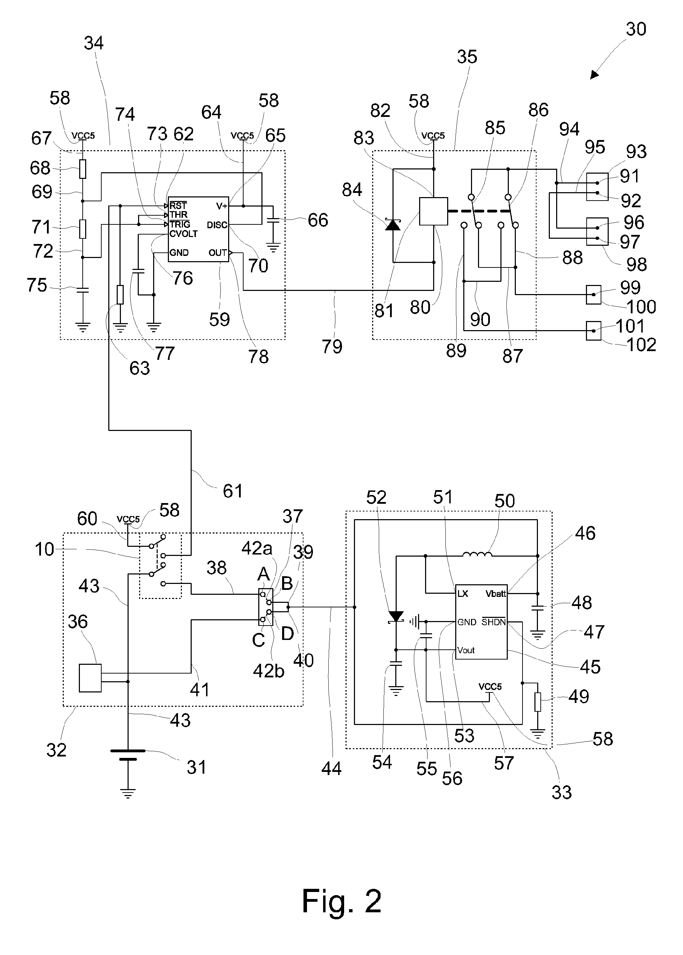

In fig. 2 a circuit diagram 30 of the first printed circuit board 11 is shown comprising a battery 31, a switch section 32, a voltage supply section 33, an oscillation generating section 34, and a relay section 35.

As mentioned above the battery 31 is an 1.5V AAA battery.

The switch section 32 comprises the slide switch 10 as well as an electrical connector 36. The switch section 32 also comprises a jumper 37. The jumper 37 is electrically connected to four electrical conductors 38,39,40,41. Initially, the electrical conductors 38,39 are electrically connected to each other via electrical conductor 42a and the electrical conductors 40,41 are electrically connected to each other via electrical conductor 42b.

The electrosurgical instrument 1 can either be in a hand-controlled mode or in a foot-controlled mode.

In the hand-controlled mode, the electrical connector 36 is electrically connected to the coagulation button 8 via the electrical wires 29a,29b and the electrosurgical instrument 1 is electrically activated for coagulation by the coagulation button 8.

In the foot-controlled mode the electrosurgical instrument 1 is electrically activated by a foot controlled switch (not shown). The foot-controlled switch (not shown) can be electrically connected to the generator 2. When the foot-controlled switch is activated, the generator 2 will apply a RF-signal to the electrosurgical instrument 1.

The electrosurgical instrument 1 is in the hand-controlled mode if the electrical conductor 42a is cut off and disconnected. The electrosurgical instrument 1 is in the foot-controlled mode if the electrical conductor 42b is cut off and disconnected.

The slide switch 10 as well as the coagulation button 8 for coagulation or cutting can each be in two positions - in the off-position (a disconnecting position) 10b or in the on-position (a connecting, activated position) 10a. The slide switch is in the off-position 10b in fig. 2.

When the slide switch 10 is in the on-position in the foot-controlled mode, voltage from the battery 31 will be connected via an electrical conductor 43, the slide switch 10, the electrical conductor 38, the electrical conductor 42a, the electrical conductor 39 and an electrical conductor 44 to the voltage supply section 33. When the coagulation button 8 is activated in the hand-controlled mode, voltage from the battery 31 will be connected via the electrical conductor 43, the electrical connector 36 and the coagulation button 8, the electrical conductor 41, the electrical conductor 42b, the electrical conductor 40 and the electrical conductor 44 to the voltage supply section 33.

The voltage supply section 33 comprises a DC-DC boost converter 45. The DC-DC boost converter can be commercially obtainable e.g. an SP6641AEK-L-5-0. The DC-DC boost converter 45 can be driven by a DC voltage between 0.9 to 4.5 V and will have DC voltage of 5.0 V as output.

The electrical conductor 44 is electrically connected directly to pins 46,47 of the DC-DC boost converter 45. The pin 46 is called Vbatt and the pin 47 is called SHDN. The pin 46 is electrically connected via a capacitor 48 to ground to remove AC signals. The pin 47 is electrically connected via a resistor 49 to ground. The electrical conductor 44 is electrically connected via an inductor 50 to a pin 51 of the DC-DC boost converter 45 called LX and the pin 51 is electrically connected via a Schottky diode 52 (STPS0520Z) to an output 53 called Vout. The cathode end of the Schottky diode 52 is electrically connected to the output 53. The output 53 is electrically connected via a capacitor 54 to ground to remove AC signals in the output signal. The output 53 is also electrically connected via a capacitor 55 to an output 56 of the DC-DC boost converter 45 called GND, which is the ground of the DC-DC boost converter 45 and electrically connected to ground. The 5.0 V DC output from the voltage supply section 33 is transmitted via an electrical conductor 57 to other parts of the circuit diagram 30. For convenience and for making it possible to have a clear overview of the circuit diagram 30 the electrical conductor 57 ends with a bar 58 and the text VCC5. The electrical conductor 57 is continued everywhere else there is a bar and the text VCC5.

The oscillation generating section 34 comprises a 555 timer IC 59 (LM555CM). When the slide switch 10 is in the on-position 10a, the voltage supply VCC5 from the DC-DC boost converter 45 is electrically connected via electrical conductors 60,61 to a pin 62 of the 555 timer IC 59 called RST. The pin 62 is electrically connected via a resistor 63 to ground.

The voltage supply VCC5 from the DC-DC boost converter 45 is electrically connected via an electrical conductor 64 to a pin 65 of the 555 timer IC 59 called V+. The pin 65 is electrically connected via a capacitor 66 to ground to remove AC signals.

The voltage supply VCC5 from the DC-DC boost converter 45 is electrically connected via an electrical conductor 67 to a resistor 68 or $R_1$ of 1kΩ. The other side of the resistor 68 seen from the electrical conductor 67 is electrically connected to an electrical conductor 69 electrically connected to a pin 70 of the 555 timer IC 59 called DISC. The electrical conductor 69 is also electrically connected to a resistor 71 or $R_2$ of 750kΩ. The other side of the resistor 71 seen from the electrical conductor 69 is electrically connected to an electrical conductor 72 electrically connected to pins 73,74 of the 555 timer IC 59 called THR and TRIG, respectively. The electrical conductor 72 is also electrically connected via a capacitor 75 or $C_1$ of 1μF to ground.

A pin 76 of the 555 timer IC 59 called CVOLT is electrically connected via a capacitor 77 to ground. The 555 timer IC 59 has also a grounded pin called GND and an output pin 78 called OUT.

When electrically connected as above the 555 timer IC 59 will when the pin 62 is high put out an oscillating signal on the output pin 78 with a frequency given by:

$$f = \left(\ln 2 \cdot C_1 \left(R_1 + 2R_2\right)\right)^{-1},$$

where $C_1$, $R_1$ and $R_2$ are as given above. In this case the frequency will be around 1 Hz. Other values of $C_1$, $R_1$ and $R_2$ are also possible giving other oscillating frequencies. To be able to adjust the pulsing frequency the resistor 68 or $R_1$ and/or the resistor 71 or $R_2$ can be potentiometers and/or the capacitor 75 or $C_1$ is a variable capacitor. The time of each pulse where the pulse is high is given by:

$$T_{High} = \ln 2 \cdot C_1 \left(R_1 + R_2\right),$$

and the time of each pulse where the pulse is low is given by:

$$T_{Low} = \ln 2 \cdot C_1 \cdot R_2 .$$

The output pin 78 is electrically connected via an electrical conductor 79 to a pin 80 of a relay 81 (IM03JR) in the relay section 35. The voltage supply VCC5 from the DC-DC boost converter 45 is electrically connected via an electrical conductor 82 to another pin 83 of the relay 81. Parallel with the relay 81 is a Schottky diode 84 (BAT54), where the anode end and the cathode end are electrically connected to the pin 80 and the pin 83, respectively.

As mentioned above, when the RST pin 62 is set high the 555 timer IC 59 will oscillate and put out an oscillating signal on the output pin 78. The oscillating signal from the output pin 78 causes the relay to flip back and forth with the same frequency as the oscillating signal and switches 85,86 will switch between electrical conductors 87,88 and 89,90, respectively.

The person skilled in the art will know that by using e.g. a Schmitt trigger or a Blocking oscillator as a timer IC it is possible to achieve an output signal, where the time of each pulse where the pulse is high is shorter than the time of each pulse where the pulse is low.

The two electrical wires 3a,3b from the electrosurgical generator 2 are electrically connected to two points of contact 91,92, respectively, in an electrical connector P1 93. Advantageously, the live connection or the phase conductor 3a from the electrosurgical generator 2 is electrically connected to the point of contact 91 and the neutral connection or return connection 3b from the electrosurgical generator 2 is electrically connected to the point of contact 92. The two points of contact 91,92 are electrically connected to electrical conductors 94,95, respectively, and the electrical conductors 94,95 are electrically connected to two points of contact 96,97, respectively, in an electrical connector P2 98. The electrical conductor 94 is also electrically connected to the two switches 85,86 and further electrically connected to either the electrical conductors 87,88 or the electrical conductors 89,90 depending on how the two switches 85,86 are positioned. The electrical conductors 87,88 are electrically connected to a point of contact 99 in an electrical connector P4 100 and the electrical conductors 89,90 are electrically connected to a point of contact 101 in an electrical connector P6 102.

When the relay flips back and forth and causes the switches 85,86 to switch between electrical conductors 87,88 and 89,90, the current from the electrosurgical generator 2 is transferred alternatively to the electrical connector P4 100 and to the electrical connector P6 102 with the same frequency as the frequency of the relay flipping.

In the hand-controlled mode, the electrical conductor 42a is cut off and the electrical input 16a of the knife switch 24 is electrically connected to the electrical connector P4 100 via the electric wire 23a. In the foot-controlled mode, the electrical conductor 42b is cut off and the electrical input 16a of the knife switch 24 is electrically connected to the electrical connector P6 102 via the electric wire 23a.

In both the hand-controlled mode and the foot-controlled mode the electrical input 16b of the knife switch 24 is electrically connected to the point of contact 96 of the electrical connector P2 98 via the electric wire 23b and the electrical input 16c of the knife switch 24 is electrically connected to the point of contact 97 of the electrical connector P2 98 via the electric wire 23c.

To use the electrosurgical instrument 1, the slide switch 10 is activated. In the foot-controlled mode the activation of the slide switch 10 will direct the voltage from the battery 31 to the voltage supply section 33 that will send a 5V DC voltage to the VCC5 58. When the slide switch 10 in the foot-controlled mode is activated the voltage VCC5 from the voltage supply section 33 is directed to the pin 62 of the 555 timer IC 59. The 555 timer IC 59 will start to oscillate when the pin 62 is high and the oscillation will pulse the signal from the RF generator 2 on the electrical connector P6 102. The knife switch 24 then applies the pulsed RF voltage on the two jaws 17a,17b for coagulating unless the cutting button 9 is activated.

In the foot-controlled mode, the electrical connector 36 and the coagulation button 8 are of no use and can advantageously be omitted. The electrosurgical instrument 1 is in the foot-controlled mode controlled by the foot-controlled switch (not shown). The foot-controlled switch can be electrically connected to the generator 2. When the foot-controlled switch is activated, the generator 2 will apply a RF-signal to the electrosurgical instrument 1.

When the slide switch 10 is in the on-position 10a, the electrosurgical instrument 1 is set to the hand-controlled mode, and the coagulation button 8 is activated, the voltage from the battery is applied on the voltage supply section 33 and the output voltage VCC5 from the voltage supply section 33 will be applied on the pin 62 of the 555 timer IC 59.

When the cutting button 9 is pressed down and the distal end 27 of the protruding member 12 will press the contact member 25 in electrical contact with the electrical contact area 26, the knife switch 24 applies the non-pulsed RF signal from the electrical connector P2 98 between the two jaws 17a,17b on one hand and the cutting member 20 on the other hand.

The person skilled in the art will know which resistances, capacitances and inductances of the resistors, capacitors and inductors mentioned above to choose to have the DC-DC boost converter 45 to deliver 5.0 V on the output 53 and to have the 555 timer IC 59 to apply an oscillating signal on the output pin 78.

Fig. 3 is an enlarged scale fragmentary view of the first printed circuit board 11 of the bipolar electrosurgical loop instrument 1 shown in fig. 1. In the first printed circuit board 11 all the electronic components shown in the circuit diagram 30 in fig. 2 are disclosed. The circuit diagram 30 in fig. 2 shows how the components are electrically connected while fig. 3 shows the physical properties of the components.

The slide switch 10 is positioned at the top of the first printed circuit board 11 in fig. 3. The electrical connector 36 connected to the coagulation button 8 is situated at the upper end of the first printed circuit board 11 in fig. 3 close to the jumper 37. The jumper 37 has two electrical conductors 42a and 42b, where the electrical conductor 42a electrically connects points A and B and the electrical conductor 42b electrically connects points C and D. One of the electrical conductors 42a and 42b must be electrically disconnected or cut for the first printed circuit board 11 to function. The electrical conductor 42a is cut if a hand-controlled mode is wanted and the electrical conductor 42b is cut if a foot-controlled mode is wanted.

Close to the electrical connector 36 connected to the coagulation button 8 and the jumper 37 are the electronic components of the voltage supply section 33, namely the Schottky diode 52, inductor 50, the capacitors 48,54,55, the DC-DC boost converter 45, and the resistor 49.

To the left of the electronic components of the voltage supply section 33 are the electronic components of the oscillation generating section 34 including the 555 timer IC 59, the capacitors 66,75,77, and the resistors 63,68,71.

At the lower part of the first printed circuit board 11 in fig. 3 is the relay section 35 comprising the relay 81 and the Schottky diode 84.

The electrical connectors 93,98,100,102 are also found on the first printed circuit board 11.

The design of the electrosurgical instrument 1 shall be understood to not be limiting for the scope of the invention.

**Claims**

1. An electrosurgical instrument (1) adapted to be used with an external electrosurgical generator (2) configured to transmit an electrical current to one or more electrodes of the electrosurgical instrument to cut or coagulate tissue by use of electrical energy from the electrode(s), **characterised in that** the electrosurgical instrument (1) incorporates a signal modificator (11) having an input end (93) electrically connectable to the electrosurgical generator (2) and an output end (98,100,102) electrically connected to one or more electrodes (17a,17b) of the electrosurgical instrument (1), where the signal modificator (11) is adapted to modify a continuous signal from the electrosurgical generator (2) into a pulsing signal to the one or more electrodes (17a,17b) of the electrosurgical instrument (1) and comprises a circuit (30) for providing the pulsing signal, wherein the signal modificator (11) is incorporated inside a handle (5) or part of a handle of the electrosurgical instrument (1).

2. An electrosurgical instrument (1) according to claim 1, **characterised in that** the circuit (30) comprises a timer IC.

3. An electrosurgical instrument (1) according to any of the preceding claims, **characterised in that** the circuit is adapted for (30) controlling switching means to electrically connect/disconnect the one or more electrodes (17a,17b,20) to/from the electrosurgical generator (2).

4. An electrosurgical instrument (1) according to any of the preceding claims, **characterised in that** the signal modificator (11) is detachably secured to the electrosurgical instrument (1).

5. An electrosurgical instrument (1) according to any of the preceding claims, **characterised in that** the circuit (30) is integrated in the handle (5) or a part of the handle, optionally said handle (5) or part of the handle is detachable.

6. An electrosurgical instrument (1) according to any of the preceding claims, **characterised in that** the pulsing signal has an interrupting frequency of between 0.1 and 10 Hz, preferably between 0.2 and 5 Hz, more preferably between 0.25 and 4 Hz, even more preferably between 0.33 and 3 Hz, furthermore preferably between 0.5 and 2 Hz, even further more preferably between 0.67 and 1.5 Hz and most preferably between 0.8 and 1.25 Hz.

7. An electrosurgical instrument (1) according to claim 6, **characterised in that** the electrosurgical instrument (1) comprises means to vary the interrupting frequency.

8. An electrosurgical instrument (1) according to any of the preceding claims, **characterised in that** the pulsing signal has a period comprising a pulse part and a quiet part, where the pulse part constitutes between 10% and 90%, preferably between 20% and 80%, more preferably between 30% and 70%, even more preferably between 40% and 60% and most preferably between 45% and 55% of the total period and the quite part constitutes the rest of the period.

9. An electrosurgical instrument (1) according to any of the preceding claims, **characterised in that** the pulsing signal has a period comprising a pulse part and a quiet part, where the pulse part constitutes between 50% and 95%, preferably between 55% and 90%, more preferably between 60% and 85%, even more preferably between 65% and 80% and most preferably between 65% and 75% of the total period and the quite part constitutes the rest of the period.

10. An electrosurgical instrument (1) according to any of the preceding claims, **characterised in that** the pulsing signal has a period comprising a pulse part and a quiet part, where the quiet part constitutes between 50% and 95%, preferably between 55% and 90%, more preferably between 60% and 85%, even more preferably between 65% and 80% and most preferably between 65% and 75% of the total period and the pulse part constitutes the rest of the period.

11. An electrosurgical instrument (1) according to any of the preceding claims, **characterised in that** the electrosurgical instrument (1) is a pair of bipolar forceps (17a,17b), a pair of bipolar forceps (17a,17b) with a cutting member (20), a bipolar loop electrode, a pair of bipolar scissors, a bipolar pencil, a bipolar scalpel, or a bipolar electrosurgical instrument incorporating and combining one or more functions thereof, or a monopolar pencil or scalpel.

12. An electrosurgical instrument (1) according to any of the preceding claims, **characterised in that** at least one of the one or more electrodes (17a,17b,20) of the electrosurgical instrument (1) has an area provided with a non-stick coating.

13. An electrosurgical apparatus comprising an electrosurgical instrument (1) according to any of the preceding claims and an electrosurgical generator (2).

**Patentansprüche**

1. Elektrochirurgisches Instrument (1), das zur Verwendung mit einem externen elektrochirurgischen Generator (2) geeignet ist, der zum Übertragen eines elektrischen Stroms auf eine oder mehrere Elektroden des elektrochirurgischen Instruments konfiguriert ist, um Gewebe durch die Verwendung von elektrischer Energie von der/den Elektrode(n) zu schneiden oder zu koagulieren, **dadurch gekennzeichnet, dass** das elektrochirurgische Instrument (1) einen Signalmodifikator (11) enthält, der ein Eingangsende (93), das elektrisch mit dem elektrochirurgischen Generator (2) verbindbar ist, und ein Ausgangsende (98, 100, 102) aufweist, das elektrisch mit der einen oder den mehreren Elektroden (17a, 17b) des elektrochirurgischen Instrumentes (1) verbunden ist, wobei der Signalmodifikator (11) eingerichtet ist, ein kontinuierliches Signal von dem elektrochirurgischen Generator (2) in ein pulsierendes Signal an die eine oder die mehreren Elektroden (17a, 17b) des elektrochirurgischen Instrumentes (1) zu modifizieren, und eine Schaltung (30) zum Bereitstellen des pulsierenden Signals umfasst, wobei der Signalmodifikator (11) in einen Handgriff (5) eingebaut oder ein Teil des Handgriffs des elektrochirurgischen Instrumentes (1) ist.

2. Elektrochirurgisches Instrument (1) nach Anspruch 1, **dadurch gekennzeichnet, dass** die Schaltung (30) ein Timer-IC aufweist.

3. Elektrochirurgisches Instrument (1) nach einem der vorhergehenden Ansprüche, **dadurch gekennzeichnet, dass** die Schaltung (30) zum Steuern einer Schalteinrichtung zum elektrischen Verbinden/Trennen von der einen oder den mehreren Elektroden (17a, 17b, 20) mit/von dem elektrochirurgischen Generator (2) eingerichtet ist.

4. Elektrochirurgisches Instrument (1) nach einem der vorhergehenden Ansprüche, **dadurch gekennzeichnet, dass** der Signalmodifikator (11) abtrennbar an dem elektrochirurgischen Instrument (1) befestigt ist.

5. Elektrochirurgisches Instrument (1) nach einem der vorhergehenden Ansprüche, **dadurch gekennzeichnet dass** der Schaltkreis (30) in den Handgriff (5) oder einen Teil des Handgriffs integriert ist, wobei gegebenenfalls der Handgriff (5) oder Teil des Handgriffs abnehmbar ist.

6. Elektrochirurgisches Instrument (1) nach einem der vorhergehenden Ansprüche, **dadurch gekennzeichnet dass** das pulsierende Signal eine Unterbrechungsfrequenz von zwischen 0,1 und 10 Hz, vorzugsweise zwischen 0,2 und 5 Hz, besonders bevorzugt zwischen 0,25 und 4 Hz, noch mehr bevorzugt zwischen 0,33 und 3 Hz, weiter vorzugsweise zwischen 0,5 und 2 Hz, sogar noch weiter bevorzugt zwischen 0,67 und 1,5 Hz und am meisten bevorzugt zwischen 0,8 und 1,25 Hz aufweist.

7. Elektrochirurgisches Instrument (1) nach Anspruch 6, **dadurch gekennzeichnet, dass** das elektrochirurgische Instrument (1) Mittel zum Variieren der Unterbrechungsfrequenz aufweist.

8. Elektrochirurgisches Instrument (1) nach einem der vorhergehenden Ansprüche, **dadurch gekennzeichnet dass** das pulsierende Signal eine Periode aufweist, die einen Impulsteil und einen ruhigen Teil umfasst, wo der Impulsteil zwischen 10 % und 90 %, vorzugsweise zwischen 20 % und 80 %, besonders bevorzugt zwischen 30 % und 70%, sogar noch mehr bevorzugt zwischen 40 % und 60 % und am meisten bevorzugt zwischen 45 % und 55 % der gesamten Periode ausmacht und der ruhige Teil den Rest der Periode bildet.

9. Elektrochirurgisches Instrument (1) nach einem der vorhergehenden Ansprüche, **dadurch gekennzeichnet dass** das pulsierende Signal eine Periode aufweist, die einen Impulsteil und einen ruhigen Teil umfasst, wo der Impulsteil zwischen 50 % und 95 %, vorzugsweise zwischen 55 % und 90 %, besonders bevorzugt zwischen 60 % und 85 %, sogar noch mehr bevorzugt zwischen 65 % und 80 % und am meisten bevorzugt zwischen 65 % und 75 % der gesamten Periode ausmacht und der ruhige Teil den Rest der Periode bildet.

10. Elektrochirurgisches Instrument (1) nach einem der vorhergehenden Ansprüche, **dadurch gekennzeichnet dass** das pulsierende Signal eine Periode aufweist, die einen Impulsteil und einen ruhigen Teil umfasst, wo der Impulsteil zwischen 50 % und 95 %, vorzugsweise zwischen 55 % und 90 %, besonders bevorzugt zwischen 60 % und 85 %, sogar noch mehr bevorzugt zwischen 65 % und 80 % und am meisten bevorzugt zwischen 65 % und 75 % der gesamten Periode ausmacht und der ruhige Teil den Rest der Periode bildet.

11. Elektrochirurgisches Instrument (1) nach einem der vorhergehenden Ansprüche, **dadurch gekennzeichnet, dass** das elektrochirurgische Instrument (1) eine bipolare Pinzette (17a, 17b), eine bipolare Pinzette (17a, 17b) mit einem Schneidelement (20), eine bipolare Schlingenelektrode, eine bipolare Schere, ein bipolarer Elektrodengriff, ein bipolares Skalpell oder ein bipolares elektrochirurgisches Instrument ist, das eine oder mehrere Funktionen davon beinhaltet oder ein monopolarer Elektrodengriff oder ein monopolares Skalpell ist.

12. Elektrochirurgisches Instrument (1) nach einem der vorhergehenden Ansprüche, **dadurch gekennzeichnet, dass** mindestens eine von der einen oder den mehreren Elektroden (17a, 17b, 20) des elektrochirurgischen Instrumentes (1) eine Fläche aufweist, die mit einer Antihaftbeschichtung versehen ist.

13. Elektrochirurgische Vorrichtung, umfassend ein elektrochirurgisches Instrument (1) nach einem der vorhergehenden Ansprüche und einen elektrochirurgischen Generator (2).

**Revendications**

1. Instrument électrochirurgical (1) adapté pour être utilisé avec un générateur électrochirurgical externe (2) configuré pour transmettre un courant électrique à une ou plusieurs électrodes de l'instrument électrochirurgical pour couper ou coaguler des tissus par l'utilisation d'énergie électrique provenant desdites une ou plusieurs électrodes, **caractérisé en ce que** l'instrument électrochirurgical (1) incorpore un modificateur de signal (11) comportant une extrémité d'entrée (93) électriquement connectable au générateur électrochirurgical (2) et une extrémité de sortie (98, 100, 102) électriquement connectée à une ou plusieurs électrodes (17a, 17b) de l'instrument électrochirurgical (1), dans lequel le modificateur de signal (11) est adapté pour modifier un signal continu provenant du générateur électrochirurgical (2) en un signal à impulsions destiné auxdites une ou plusieurs électrodes (17a, 17b) de l'instrument électrochirurgical (1) et comprend un circuit (30) pour fournir le signal à impulsions, dans lequel le modificateur de signal (11) est incorporé à l'intérieur d'une poignée (5) ou d'une partie de poignée de l'instrument électrochirurgical (1).

2. Instrument électrochirurgical (1) selon la revendication 1, **caractérisé en ce que** le circuit (30) comprend un circuit intégré à minuterie.

3. Instrument électrochirurgical (1) selon l'une quelconque des revendications précédentes, **caractérisé en ce que** le circuit (30) est adapté pour contrôler un moyen de commutation pour connecter/déconnecter électriquement lesdites une ou plusieurs électrodes (17a, 17b, 20) vers/depuis le générateur électrochirurgical (2).

4. Instrument électrochirurgical (1) selon l'une quelconque des revendications précédentes, **caractérisé en ce que** le modificateur de signal (11) est fixé de manière détachable à l'instrument électrochirurgical (1).

5. Instrument électrochirurgical (1) selon l'une quelconque des revendications précédentes, **caractérisé en ce que** le circuit (30) est intégré dans la poignée (5) ou dans une partie de la poignée, et **en ce que** ladite poignée (5) ou partie de poignée est éventuellement détachable.

6. Instrument électrochirurgical (1) selon l'une quelconque des revendications précédentes, **caractérisé en ce que** le signal à impulsions a une fréquence d'interruption comprise entre 0,1 et 10 Hz, de préférence entre 0,2 et 5 Hz, de préférence encore entre 0,25 et 4 Hz, de préférence encore entre 0,33 et 3 Hz, de préférence encore entre 0,5 et 2 Hz, de préférence encore entre 0,67 et 1, 5 Hz, et de manière préférée entre toutes entre 0,8 et 1,25 Hz.

7. Instrument électrochirurgical (1) selon la revendication 6, **caractérisé en ce que** l'instrument électrochirurgical (1) comprend un moyen permettant de faire varier la fréquence d'interruption.

8. Instrument électrochirurgical (1) selon l'une quelconque des revendications précédentes, **caractérisé en ce que** le signal à impulsions présente une période comprenant une partie d'impulsion et une partie de repos, où la partie d'impulsion constitue entre 10 % et 90 %, de préférence entre 20 % et 80 %, de préférence encore entre 30 % et 70 %, de préférence encore entre 40 % et 60 %, et de manière préférée entre toutes entre 45 % et 55 % de la période totale, et la partie de repos constitue le reste de la période.

9. Instrument électrochirurgical (1) selon l'une quelconque des revendications précédentes, **caractérisé en ce que** le signal à impulsions présente une période comprenant une partie d'impulsion et une partie de repos, où la partie d'impulsion constitue entre 50 % et 95 %, de préférence entre 55 % et 90 %, de préférence encore entre 60 % et 85 %, de préférence encore entre 65 % et 80 %, et de manière préférée entre toutes entre 65 % et 75 % de la période totale, et la partie de repos constitue le reste de la période.

10. Instrument électrochirurgical (1) selon l'une quelconque des revendications précédentes, **caractérisé en ce que** le signal à impulsions présente une période comprenant une partie d'impulsion et une partie de repos, où la partie de repos constitue entre 50 % et 95 %, de préférence entre 55 % et 90 %, de préférence encore entre 60 % et 85 %, de préférence encore entre 65 % et 80 %, et de manière préférée entre toutes entre 65 % et 75 % de la période totale, et la partie d'impulsion constitue le reste de la période.

11. Instrument électrochirurgical (1) selon l'une quelconque des revendications précédentes, **caractérisé en ce que** l'instrument électrochirurgical (1) est une paire de forceps bipolaires (17a, 17b), une paire de forceps bipolaires (17a, 17b) avec un élément de coupe (20), une électrode à boucle bipolaire, une paire de ciseaux bipolaires, un crayon bipolaire, un scalpel bipolaire, ou un instrument électrochirurgical bipolaire incorporant et combinant une ou plusieurs fonctions de ces instruments, ou un crayon ou scalpel monopolaire.

**12.** Instrument électrochirurgical (1) selon l'une quelconque des revendications précédentes, **caractérisé en ce qu'**au moins une desdites une ou plusieurs électrodes (17a, 17b, 20) de l'instrument électrochirurgical (1) présente une surface pourvue d'un revêtement antiadhésif.

**13.** Appareil électrochirurgical comprenant un instrument électrochirurgical (1) selon l'une quelconque des revendications précédentes et un générateur électrochirurgical (2).

Fig. 1

Fig. 2

Fig. 3

**EP 2 606 845 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 2160993 A
- WO 2010108523 A
- EP 1150616 A
- US 5478347 A